# EUROPÄISCHE PATENTSCHRIFT

(11) **EP 0 706 526 B1**
(45) Veröffentlichungstag und Bekanntmachung des Hinweises auf die Patenterteilung: **17.09.2003**
(21) Anmeldenummer: 94918743.9
(22) Anmeldetag: 28.06.1994
(51) Int. Cl.: C07D 491/04, G03C 1/73

(54) **PHOTOCHROME VERBINDUNGEN**
PHOTOCHROMIC COMPOUNDS
COMPOSES PHOTOCHROMIQUES

(30) Priorität: 28.06.1993 DE 4321487
(43) Veröffentlichungstag der Anmeldung: 17.04.1996
(73) Patentinhaber: Rodenstock GmbH, 80469 München (DE)
(72) Erfinder: MELZIG, Manfred, D-82234 Wessling (DE); ZINNER, Herbert, D-93080 Pentling (DE)
(74) Vertreter: Münich, Wilhelm, Dr.
(86) Internationale Anmeldenummer: DE9400737
(87) Internationale Veröffentlichungsnummer: WO95000519

(56) Entgegenhaltungen:
- EP-A- 0 250 193
- FR-A- 1 450 583
- FR-A- 1 451 332
- FR-A- 2 093 370
- US-A- 3 567 605
- CHEMICAL ABSTRACTS, vol. 118, no. 25, 1993, Columbus, Ohio, US; abstract no. 254269e, V.V. NIKOLAEVA ET AL. 'Photochromic spiropyrans of coumarin series' Seite 817 ; & KHIM. GETEROTSIKL. SOEDIN 1992, (5), 601-4

## Beschreibung

### Technisches Gebiet

Die Erfindung bezieht sich auf die Verwendung einer photochromen Verbindung als Einzelsubstanz.

### Stand der Technik

Derartige photochrome Verbindungen sind beispielsweise Pyrane, wie sie u.A. in der US-PS 5,066,818, der PCT-Veröffentlichung WO 92/09593 oder der US-PS 4,818,096 beschrieben sind, oder Oxazine, wie sie u.a. in den US-PSen 3,652,172, 3,578,602, 4,215,010 und 4,637,698 beschrieben sind.

Ferner sind aus der FR-A-1,450,583 und der FR-A-1,451,332 Benzole und Naphthaline, die über zwei ankondensierte Spiropyrane mit Indolin verknüpft sind, sowie Systeme bekannt, bei denen zwei identische Indolino-spirobenzopyrane über Phenyl-Phenyl oder Phenyl-CH2-Phenylbrücken verknüpft sind. Die FR-A-2.093.370 beschreibt bifunktionelle Spiropyrane, die über Alkylreste miteinander verbunden sind. Weitere Synthesen mit verknüpften Indolinspiropyraneinheiten wurden in den 60er Jahren von NCR, Dayton, Ohio durchgeführt.

Die bekannten Pyrane und Oxazine absorbieren jeweils Licht in einem vergleichsweise engen Spektralbereich. Dies hat zur Folge, dass Gegenstände, wie beispielsweise Brillengläser oder Sonnenschutzdächer, die mit einer dieser photochromen Verbindungen eingefärbt sind, intensiv beispielsweise blau eingefährbt sind. In einer Reihe von Anwendungsfällen wäre es jedoch wünschenswert, wenn der mit der photochromen Verbindung eingefärbte Gegenstand eine neutral graue oder braune Farbe hätte.

Derzeit behilft man sich damit, dass man zwei oder mehr photochrome Farbstoffe verwendet, die in unterschiedlichen Wellenlängenbereichen absorbieren. Hierzu wird auf die EP-A-O 146 136, die EP-A-0 397 803 oder die EP-A-0 250 193 verwiesen.

Diese Vorgehensweise hat jedoch den Nachteil, dass das Aufhellungs- und das Abdunkelungsverhalten der verwendeten photochromen Verbindungen genau aufeinander abgestimmt sein müssen. Ansonsten würde der eingefärbte Gegenstand während der Aufhellung und/oder der Abdunkelung seine Farbe ändern. Darüberhinaus müssen die Farbstoffe mit einem in engen Grenzen tolerierten stöchiometrischen Verhältnis eingebracht werden.

### Darstellung der Erfindung

Der Erfindung liegt die Aufgabe zugrunde, die vorstehend aufgezeigten Nachteile des Standes der Technik dadurch zu vermeiden, dass die Verwendung einer photochromen Verbindung als Einzelsubstanz zur photochromen Einfärbung eines durchsichtigen Elementes aus einem Kunststoffmaterial, das im angeregten Zustand eine neutral graue oder braune Farbe aufweist, angegeben wird, so dass die Stöchiometrie der einzelnen photochromen Teilsysteme immer gewährleistet ist. Zudem soll durch die erfindungsgemäße Vorgehensweise ein verbessertes Aufhellungs- und Abdunkelungsverhalten verglichen mit der Verwendung der gleichen photochromen Substanzen als Einzelsubstanzen erreicht werden.

Besonders bevorzugt ist es, wenn die Moleküle der erfindungsgemäßen photochromen Verbindungen zwei oder mehrere photochrome Pyran-, Oxazin- oder Pyran- und Oxazineinheiten tragen. Dabei ist wiederum von besonderem Vorteil, wenn die photochromen Teilsysteme 2H-Pyrane und/oder Spirooxazine sind. Als Pyran- und Oxazineinheiten können prinzipiell bekannte Pyrane und Oxazine verwendet werden.

Die erfindungsgemäßen Moleküle mit 2H-Pyranen und/oder Spirooxazinen haben nämlich die Eigenschaft, daß sie durch ihre hochkondensierten Ringsysteme längerwellig als die Einzelsysteme nach dem Stand der Technik absorbieren.

Da damit auch der längerwellige UV-Anteil der Sonnenstrahlung, der zugleich intensiver ist, zur Anregung herangezogen werden kann, werden bei gleicher Farbstoffkonzentration wesentlich intensivere Eindunkelungen, z.B. für Sonnenbrillen, erreicht.

### Darstellung eines Ausführungsbeispiels

Im folgenden wird exemplarisch der Aufbau einen erfindungsgemäß verwendeten Verbindung beschrieben.

Hierzu wird auf das beigefügte Diagramm Bezug genommen, das ein Beispiel für die Transmission einer erfindungsgemäß verwendeten Verbindungen in Abhängigkeit von der Wellenlänge zeigt.

Erfindungsgemäß verwendete Verbindungen können beispielsweise durch Kondensation von substituierten Polyhydroxyaromaten mit den entsprechenden Reaktionspartnern in aufeinanderfolgenden Schritten erhalten werden.

Der hier von 2,6-Dihydroxynaphthalin ausgehenden Synthesewege soll nur als Beispiel dienen. Auch 2,3-, 1,6- und 2,7-Dihydroxynaphthaline sind mit guten Ausbeuten einsetzbar. In gleicher Weise können andere In gleicher Weise können Polyhydroxyaromaten, Derivate des Benzols, Biphenyls, Anthracens oder Phenanthrens verwendet werden.

Alle können darüberhinaus weitere funktionelle Gruppen tragen. Wichtig ist einzig, dass mindestens zwei Hydroxygruppen des Moleküls jeweils mindestens eine freie ortho-Stellung besitzen. Auf Wegen, die eine Nitrosierung oder eine Umlagerung beinhalten, muß die para-Position zur Hydroxygruppe, beispielsweise durch Cl oder Br besetzt sein.

Als Reaktionspartner sind im Falle der Acetylide neben Diphenylcarbinolacetyliden auch entsprechende Verbindungen geeignet, bei denen die beiden Phenylringe starr verknüpft sind, beispielsweise Anthracenderivate. Auch nicht aromatische Acetylide, wie die des Norbornans oder Adamantans sind verwendbar. Im Falle einer Nitrierung des Hydroxyaromaten mit Methylenbasen sind alle eine aktive Methylengruppe tragenden und spiroverknüpfenden Systeme brauchbar, beispielsweise 2-Methylenbenzoxazole, -benzothiazole etc. Falls sie in Form ihrer Salze eingesetzt werden, müssen mindestens molare Mengen an Kondensationsmittel eingesetzt werden.

### Versuchsbeschreibung:

Nachstehend wird eine Syntheseroute beschrieben, die von Dihydroxynaphthalinen ausgeht. Im Prinzip sind aber auch andere Aromaten, wie Benzol, Biphenyle, Anthracene oder Phenanthrene verwendbar, sofern sie an einem oder mehreren ihrer Ringe insgesamt mindestens 2 Hydroxygruppen mit je mindestens einer freien ortho-Position besitzen. Sowohl die Auswahl des Polyhydroxyaromaten, als auch die der Reaktionspartner bestimmen die Merkmale des Endprodukts. Verwendet man 2,6-Naphthalindiol als Ausgangsprodukt, so sind durch Umsetzung zunächst nur einer Hydroxygruppe mit einem Carbinol C1, evtl. Isolierung des Zwischenprodukts und nachfolgende Umsetzung mit einem anderen Carbinol C2 reine Dipyrano-naphthaline erhältlich. Diese absorbieren längerwellig als die Monoprodukte, angeregt werden goldorange bis zinnoberrote Farbtöne erhalten.

In analoger Weise lassen sich auch andere Naphthalindiole, insbesondere 2,7-, 2,3-, 1,5- und 1,6-Dihydroxynaphthalin, und 2,6-Dihydroxyanthracen zu zwei photochrome Einheiten tragenden Molekülen umsetzen. Hierbei gelten für die beiden photochromen Teilsysteme die von den Einzelsysteme her bekannten Eigenschaften. Beispielsweise ergeben sich aus der Verwendung von Hydroxyfunktionen in 1, 4, 5 oder 8-Stellung des Naphthalins oder Anthracens Verbindungen, die gegenüber den 2, 3, 6 oder 7-Hydroxyaromaten angeregt ca. 30 nm längerwellig absorbieren und deutlich langsamer aufhellen. Ebenso gilt die aus der US 5,066,818 her bekannte Reduzierung der Aufhellgeschwindigkeit bei Substitution der Arylsubstituenten (in 2-Stellung des Pyranrings) in ortho-Stellung zur Verknüpfung oder die bathochrome Verschiebung der photochromen Farbe bei Substitution des Naphthalinsystems in 3-Stellung oder in 6-Stellung (US 5,238,981). In gleicher Weise bleiben die Vorzüge der in 3-Stellung des Naphthalinrings substituierten Spiro(indolin-naphthoxazine) (WO 92/09593) erhalten.

Gegenüber diesen aus dem Stand der Technik her bekannten Verbindungen ergibt sich eine bathochrome Verschiebung der Absorption im nicht angeregten Zustand. Gleichzeitig steigt der Extinktionskoeffizient der längstwelligen Absorption. Dadurch ergibt sich beispielsweise bei Dipyrano-Systemen eine gegenüber einer Mischung der Einzelkomponenten erheblich bessere Effizienz bei Bestrahlung mit natürlichem Sonnenlicht.

### Synthesen:

### Herstellung des Carbinols 1:

54,7 g (0,30 mol) Benzophenon werden in 150 ml trockenem Dimethylsulfoxid gelöst, zu der Lösung werden unter Rühren 27,6 g (0,30 mol) Lithiumacetylid-Ethylendiamin-Komplex zugetropft. Die Lösung färbt sich dunkelgelb, wobei ein feiner, heller Niederschlag entsteht. Die Mischung wird bei Raumtemperatur noch 16 h gerührt. Die orangegelbe Suspension wird auf 750 g zerstoßenes Eis gegossen und sofort mit 2n HCl sauer eingestellt. Die hellockerfarbene Suspension wird mit Ether solange ausgeschüttelt, bis sich der an der Phasengrenze absetzende Niederschlag völlig im Ether aufgelöst hat. Die vereinigten Etherextrakte werden mit Na2SO4 getrocknet und filtriert. Dem Filtrat wird der Ether am Rotationsverdampfer entzogen. Zurück bleiben 55,2 g eines orangen Öles, das über Nacht auskristallisiert. Es wird mittels NMR-Daten als 1,1 Diphenyl-2-propin-1-ol charakterisiert.

### Herstellung des Carbinols 2:

64 g (0,48 mol) wasserfreies Aluminiumchlorid wird in 160 ml trockenem 1,2-Dichlorethan unter Rühren suspendiert, dann werden bei unter 20°C 65g (0,42 mol) o-Toluylsäurechlorid zugetropft. Unter Auflösung der Al-verbindung färbt sich die Suspension dunkler. Bei weiterhin unter 20°C werden anschließend 43,2 g (0,40 mol) Anisol zugetropft. Die Lösung färbt sich dunkelbraun, wird noch 1 h bei Raumtemperatur gerührt und 15 h stehen gelassen. Man gießt vorsichtig auf 300 g zerstoßenes Eis, säuert mit 6 n HCl leicht an und trennt die organische Phase im Scheidetrichter ab. Sie wird mit 2%iger NaOH-lösung mehrmals ausgeschüttelt, mit Natriumsulfat getrocknet und filtriert. Nach dem Abtrennen des Lösemittels verbleiben 70,2 g eines orangen Öles. Aus Ether/Hexan umkristallisiert wurde die Verbindung als 2-Methyl-4'-methoxy-benzophenon identifiziert. Dieses wird in 150 ml trockenem Dimethylsulfoxid gelöst, zu der hellorangen Lösung werden unter Rühren 28,5 g (0,31 mol) Lithiumacetylid-Ethylendiamin-Komplex zugetropft. Die Lösung färbt sich dunkler. Die dunkelbraune Suspension wird wie unter a) weiterbearbeitet und ergibt 76,9 g eines dunkel-orangen Öles. Die NMR-Analyse der aus Pentan/Ether umkristallisierten Verbindung bestätigte die Substanz als 1-(2-Methylphenyl)-1-(4-Methoxyphenyl)-2-propin-1-ol.

### Synthese der photochromen Verbindung:

Zu 16,0 g (0,10 mol) 2,6-Dihydroxynaphthalin in 150 ml Toluol gibt man eine Spatelspitze Toluol-4-sulfonsäure. Unter Rühren tropft man bei Raumtemperatur hierzu 20,8 g (0,10 mol) des Carbinols 1 in 40 ml Toluol, wobei sich die Lösung sofort dunkel färbt und orangerote Photochromie zeigt. Man erhitzt auf 60°C und rührt noch 1 h nach. Zu dieser Lösung tropft man dann 25,2 g (0,10 mol) des Carbinols 2 in 60 ml Toluol. Die Lösung wird noch dunkler und zeigt karminrote Photochromie. Man hält die Temperatur noch 30 min bei 60°C. Nach dem Abkühlen schüttelt man zur Entfernung unumgesetzten Ausgangsprodukts noch zweimal mit 300 ml 5%iger NaOH aus. Die abgetrennte organische Phase wird mit Natriumsulfat getrocknet und filtriert. Dem Filtrat wird das Toluol am Rotationsverdampfer entzogen. Der Rückstand wird mit Methylenchlorid aufgenommen und an Aluminiumoxid chromatographiert. Die erste Fraktion, die rote Photochromie zeigt, wird gesammelt. Nach dem Abziehen des Laufmittels erhält man 28,6 g eines dunkelroten Öls. Dieses kann durch nochmalige Chromatographie mit Toluol/Hexan in die 2 symmetrischen und das gewünschte Produkt, das eine etwas höhere Retentionszeit besitzt, getrennt werden. Durch Umkristallisation erhält aus Ether/Hexan erhält man 16,3 g schwach gelbliche Kristalle. Mittels NMR-Daten wurde die Substanz als 2,2-Diphenyl-8-(2-methylphenyl)-8-(4-methoxyphenyl)-dioxa(1,7)chrysen identifiziert :

### Herstellung der Polymerproben:

Da die erfindungsgemäßen Verbindungen vor allem für den Einsatz in Kunststoffmaterialien, insbesondere für den Einsatz in Brillengläsern, entwickelt wurden, ist ihr Verhalten in diesen Matrices von ausschlaggebender Bedeutung. Aufgrund ihrer unterschiedlichen Struktur, Raumerfüllung und Polarität zeigen die erfindungsgemäßen Substanzen und die entsprechenden Verbindungen nach dem Stand der Technik bei Färbungen von Kunststoffmaterialien nach den üblichen Verfahren der Oberflächenfärbung ein unterschiedliches Diffusions- bzw. Migrationsverhalten. Zudem kann die exakte Konzentration des Farbstoffs im Kunststoff nicht angegeben werden, so dass zwischen den Verbindungen und ihrer Absorptionsintensität, resp. ihrem molaren Extinktionskoeffizienten keine Relation hergestellt werden kann. Aus diesem Grunde wurde für den Vergleich auf ein anderes Verfahren zur photochromen Einfärbung von Kunststoffmaterialien zurückgegriffen, das diese Nachteile nicht zeigt.

Es ist exemplarisch in der EP 0 227 337 beschrieben. Hier wurde ein handelsübliches Monomer (TS-150, Tokuyama Soda) mit jeweils 500 ppm der photochromen Verbindungen versetzt, in 2 mm Planglasformen gegossen und entsprechend der Herstellervorschriften polymerisiert. Diese Proben wurden in einem hochauflösenden Spektralphotometer (Lambda 9, Perkin Elmer) vermessen.

### Ergebnisse:

Das Diagramm zeigt entsprechend die Absorption des Beispiels. Die längstwellige Absorptionsbande fällt durch ihre bathochrome Verschiebung und ihre sehr hohe Intensität auf.

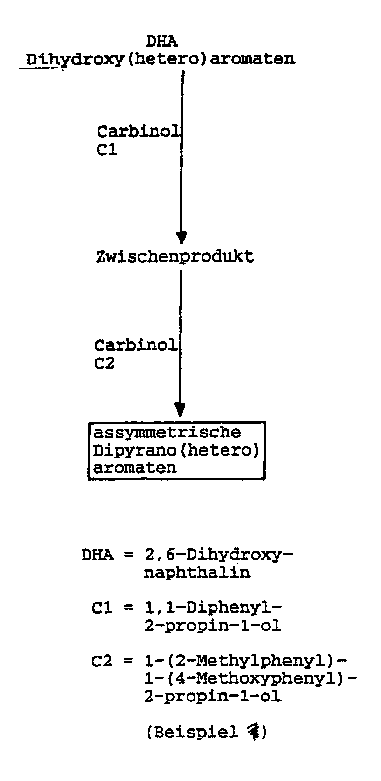

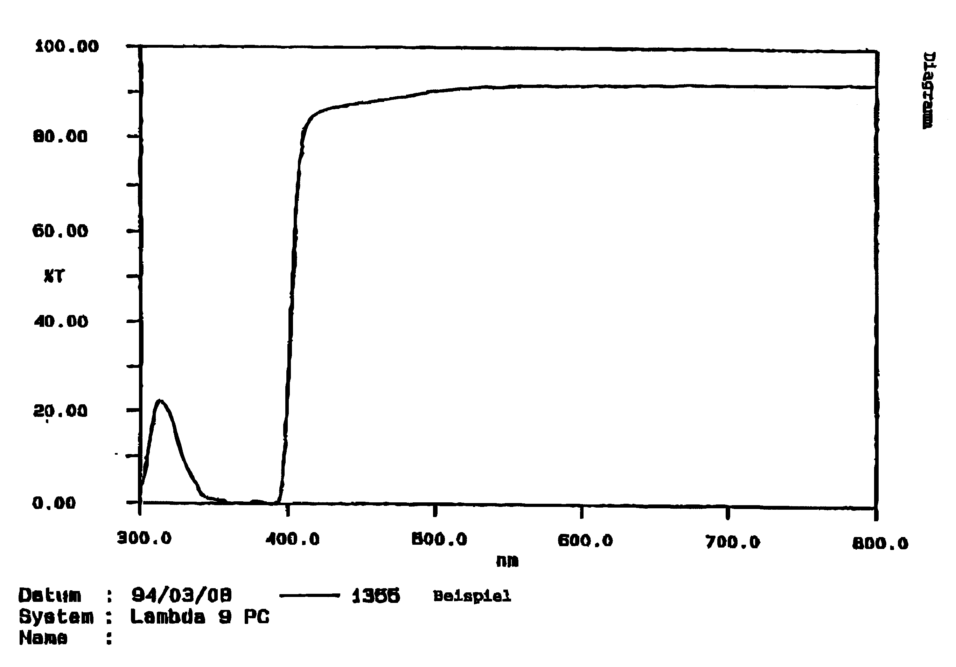

## Patentansprüche

1. Verwendung einer photochromen Verbindung als Einzelsubstanz zur photochromen Einfärbung eines durchsichtigen Elementes aus einem Kunststoffmaterial, das im angeregten Zustand eine neutral graue oder braune Farbe aufweist, wobei
- die photochrome Verbindung aus einem Aromaten mit mindestens zwei, nicht identischen, an den Aromat ankondensierten photochromen Teilsystemen besteht,
- der Aromat ausgewählt ist aus Benzol, Biphenyl, Naphthalin, Anthracen und Phenanthren, und
- wenigstens zwei photochrome Teilsysteme aus Oxazinen und/oder Pyranen mit Ausnahme von Indolinospiropyran derart ausgewählt sind,
dass aufgrund der unterschiedlichen Absorptionswellenlängen der angeregten photochromen Teilsysteme das durchsichtige Element im angeregten Zustand der photochromen Verbindung eine neutral graue oder braune Farbe hat, und
dass die Kondensationspunkte dem jeweiligen Sauerstoffatom des Pyrans bzw. dem Sauerstoff- und Stickstoffatom des Oxazins unmittelbar benachbart sind, so dass sich eine bathochrome Verschiebung der Absorption von Teilsystemen im nicht angeregten Zustand gegenüber den jeweiligen Einzelsystemen ergibt.

2. Verwendung einer photochromen Verbindung nach Anspruch 1, bei der die photochromen Teilsysteme 2H-Pyrane und/oder Spirooxazine sind.

## Claims

1. Use of a photochromic compound as single substance for photochromic pigmentation of a transparent element made of a plastic material which, in the excited state, has a neutral grey or brown colour,
- the photochromic compound comprising an aromatic with at least two, non-identical, photochromic partial systems which are condensed onto the aromatic,
- the aromatic being selected from benzene, biphenyl, naphthalene, anthracene and phenanthrene, and
- at least two photochromic partial systems made of oxazines and/or pyrans with the exception of indolinospiropyran being selected such that,
because of the different absorption wavelengths of the excited photochromic partial systems, the transparent element in the excited state of the photochromic compound has a neutral grey or brown colour, and
the condensation points are directly adjacent to the respective oxygen atom of the pyran or to the oxygen and nitrogen atom of the oxazine, so that there is produced a bathochromic displacement of the absorption of partial systems in the non-excited state relative to the respective single systems.

2. Use of a photochromic compound according to claim 1, in which the photochromic partial systems are 2H-pyrans and/or spirooxazines.

## Revendications

1. Utilisation d'un composé photochrome comme substance unique pour la coloration photochrome d'un élément transparent en matière plastique, élément transparent qui montre à l'état excité une couleur gris neutre ou brun neutre, dans laquelle
- le composé photochrome consiste en un aromatique avec au moins deux sous-systèmes photochromes non-identiques condensés sur l'aromatique,
- l'aromate est choisi dans le groupe comprenant le benzène, le biphényle, le naphtalène, l'anthracène, le phénanthrène, et
- au moins deux sous-systèmes photochromes sont choisis dans le groupe comprenant les oxazines et/ou les pyranes à l'exception des indolino-spiropyranes de telle sorte que
en raison des différentes longueurs d'absorption des sous-systèmes photochromes excités, l'élément transparent a une couleur gris neutre ou brun neutre à l'état excité du composé photochrome, et que
les points de condensation avoisinent directement l'atome d'oxygène respectif du pyrane ou l'atome d'oxygène et l'atome d'azote de l'oxazine, tel qu'il en résulte un déplacement bathochrome de l'absorption des sous-systèmes à l'état non-excité par rapport aux systèmes isolés respectifs.

2. Utilisation d'un composé photochrome selon la revendication 1, dans laquelle les sous-systèmes photochromes sont des 2H-pyranes et/ou des spirooxazines.
